# EUROPEAN PATENT APPLICATION

(11) **EP 1 300 152 A1**
(43) Date of publication of application: **09.04.2003**
(21) Application number: 01932244.5
(22) Date of filing: 24.05.2001
(51) Int. Cl.: A61K 31/573, A61K 9/70, A61K 47/16, A61K 47/10, A61K 47/18, A61K 47/14, A61K 47/12, A61K 47/34

(54) **PERCUTANEOUS ABSORPTION TYPE STEROID PREPARATION FOR EXTERNAL USE**

(30) Priority: 31.05.2000 JP 2000161448
(71) Applicant: Nichiban Company Limited, Bunkyo-ku, Tokyo 112-8663 (JP); Kuraray Co., Ltd., Kurashiki-shi Okayama-ken 710-0801 (JP); TAIHO PHARMACEUTICAL COMPANY LIMITED, Chiyoda-ku, Tokyo 101-8444 (JP)
(72) Inventor: FURUKAWA, Yoshiko, c/o NICHIBAN CO., LTD., Tokyo 112-8663 (JP); NAKAO, Ken-ichi, c/o NICHIBAN CO., LTD., Tokyo 112-8663 (JP); KOKUBO, Takemasa, c/o NICHIBAN CO., LTD., Tokyo 112-8663 (JP); KANEHIRA, Koichi, c/o KURARAY CO., LTD., Kurashiki-shi, Okayama 710-0801 (JP)
(74) Representative: Jones, Helen Marjorie Meredith
(86) International application number: JP0104359
(87) International publication number: WO01091757

(57) **Abstract**

A percutaneous absorption type steroid preparation for external use, wherein the preparation form of the preparation for external use is a patch that a pressure-sensitive adhesive layer containing a steroidal antiphlogistic is provided on a support, and the pressure-sensitive adhesive layer further contains a dissolution aid for the steroidal antiphlogistic and an accelerator for percutaneous absorption.

## Description

### TECHNICAL FIELD

The present invention relates to a percutaneous absorption type steroid preparation for external use, and more particularly to a percutaneous absorption type steroid preparation for external use for percutaneously administering a steroidal antiphlogistic to a local tissue.

### BACKGROUND ART

Examples of commercially available percutaneous absorption type preparation for external use containing a nonsteroidal antiphlogistic include those of various preparation forms such as gel, ointment, cream, poultice and plaster. These percutaneous absorption type nonsteroid preparations for external use have physiological activity such as anti-inflammatory action, anti-allergic action and immunosuppressive action and exhibit a local anti-inflammatory effect, but can not exhibit a sufficient anti-inflammatory effect on a disease in a deep part of a vital tissue, such as rheumatoid arthritis, osteoarthritis or tendosynovitis.

On the other hand, a steroidal antiphlogistic cannot exhibit an anti-inflammatory effect even when it is applied to a diseased part as a preparation for external use, such as an ointment because it does not deeply penetrate into the interior of the skin. For this reason, a disease in a deep part of a vital tissue, such as rheumatoid arthritis has heretofore been mainly treated by internal use and/or injection of a steroidal antiphlogistic. However, the internal use and injection of the steroidal antiphlogistic have involved a problem that the steroidal antiphlogistic itself has strong side effects.

Accordingly, Japanese Patent Application Laid-Open No. 61-229824 has proposed a percutaneous absorption type steroid preparation for external use obtained by using a substituted steroid which has been enhanced in permeability through the skin and transmigration in the interior of a vital body by replacing the OH group at a 21-position of a steroidal antiphlogistic by esterification reaction or the like and formulating it into a preparation form such as ointment, cream or jelly. More specifically, Examples of this publication shows examples of gel ointments prepared by mixing a substituted steroid such as prednisolone monosuccinate with ethanol, polyethylene glycol, a gelling agent and water.

Preparations for external use containing such a steroidal antiphlogistic, such as gel ointments can exhibit a local anti-inflammatory effect on rheumatoid arthritis, tendosynovitis, etc. by applying them to a diseased part. However, the percutaneous absorption type steroid preparations for external use, such as gel ointments are difficult to precisely control a dose of the steroidal antiphlogistic because they are applied by coating the skin with a proper amount of the preparation, and moreover to avoid development of systemic side effects after applied once.

### DISCLOSURE OF THE INVENTION

It is an object of the present invention to provide a novel percutaneous absorption type steroid preparation for external use which contains a steroidal antiphlogistic, can precisely control a dose of the steroidal antiphlogistic, is good in percutaneous absorption and can easily avoid a side effect if the side effect develops after application.

The present inventors have paid attention to the feasibility of a patch containing a steroidal antiphlogistic as a means for overcoming the therapeutic problems of the conventional external preparations such as gel ointments containing the steroidal antiphlogistic. More specifically, the present inventors have produced a patch by providing a pressure-sensitive adhesive layer containing a steroidal antiphlogistic on a support to test its pharmacological effect. As a result, it has been found that the permeability of the steroidal antiphlogistic through the skin is markedly deteriorated, and the pharmacological effect cannot reach a necessary level.

More specifically, it has heretofore been indicated that when the steroidal antiphlogistic is formulated into a preparation for external use, there arises a problem that the permeability through the skin is poor. However, the mere production of the patch makes this problem more and more serious. Even if a modified steroidal antiphlogistic is used for the purpose of improving the permeability through the skin and penetrability into the interior of the skin, the permeability through the skin is markedly deteriorated when such an antiphlogistic is contained in a pressure-sensitive adhesive layer.

The present inventors have thus carried out a further research. As a result, it has been found that a dissolution aid for the steroidal antiphlogistic and an accelerator for percutaneous absorption should be further contained together with the steroidal antiphlogistic in the pressure-sensitive adhesive layer of the patch, whereby the permeability of the steroidal antiphlogistic through the skin is markedly improved to provide a preparation for external use exhibiting a satisfactory pharmacological effect.

Since the preparation form of this preparation for external use is a patch that a pressure-sensitive adhesive layer containing a prescribed amount of a steroidal antiphlogistic is provided on a support, the dose of the steroidal antiphlogistic can be precisely controlled. In addition, when a side effect manifests, the side effect can be avoided by immediately peeling the patch from the surface of the skin. The present invention has been led to completion on the basis of these findings.

According to the present invention, there is thus provided a percutaneous absorption type steroid preparation for external use comprising a steroidal antiphlogistic, wherein
(1) the preparation form of the preparation for external use is a patch that a pressure-sensitive adhesive layer containing the steroidal antiphlogistic is provided on a support, and
(2) the pressure-sensitive adhesive layer further contains a dissolution aid for the steroidal antiphlogistic and an accelerator for percutaneous absorption.

### BEST MODE FOR CARRYING OUT THE INVENTION

### (1) Support:

The support used in the present invention is preferably a film (including a sheet), nonwoven fabric, fabric or the like having moderate stretchability and flexibility from the viewpoints of easy application to the surface of the skin, follow-up property to the movement of the skin surface, etc.

As examples of a material of the film used as the support include polyolefin resins such as polyethylene and polypropylene; polyacrylic resins such as polymethyl methacrylate and polyethyl methacrylate; polyester resins such as polyethylene terephthalate, polybutylene terephthalate and polyethylene naphthalate; and besides cellophane, polyvinyl alcohol, ethylene-vinyl alcohol copolymers, polyvinyl chloride, polystyrene, polyurethane, polyacrylonitrile, fluororesins, styrene-isoprene-styrene copolymers, styrene-butadiene rubber, polybutadiene, ethylene-vinyl acetate copolymers, polyamide and polysulfone.

As examples of a material of the nonwoven fabric include polyolefin resins such as polyethylene and polypropylene; polyester resins such as polyethylene terephthalate, polybutylene terephthalate and polyethylene naphthalate; and besides rayon, polyamide, poly(ester ethers), polyurethane, polyacrylic resins, polyvinyl alcohol, styrene-isoprene-styrene copolymers and styrene-ethylene-propylene-styrene copolymers. As examples of a material of the fabric include cotton, rayon, polyacrylic resins, polyester resins and polyvinyl alcohol.

### (2) Steroidal antiphlogistic:

Examples of the steroidal antiphlogistic used in the present invention include cortisone, hydrocortisone, prednisolone, methylprednisolone, triamcinolone, dexamethasone, betamethasone, paramethasone, and derivatives and/or salts thereof.

As examples of the derivatives, may be mentioned substitution products obtained by esterification, such as succinates, oxalates, malonates, glutarates and adipates; products substituted by a farnesyl group; and salts of these products. Examples of the salts include the sodium salts, potassium salts, tartaric acid salts, citric acid salts, maleic acid salts and fumaric acid salts.

Among these, prednisolone, prednisolone derivatives such as 11β,17α,21-trihydroxy-1,4-pregnadiene-3,20-dione 21-[(E,E)-3,7,11-trimethyl-2,6,10-dodecatrienoate] [hereinafter referred to as "prednisolone 21-(2E,6E-farnesylate)] and prednisolone monosuccinate and/or salts thereof are preferred.

### (3) Dissolution aid:

The dissolution aid is a substance which fills the action of enhancing the solubility of the steroidal antiphlogistic upon preparation of a coating formulation containing the respective components such as a pressure-sensitive adhesive and preventing deposition of the steroidal antiphlogistic in the pressure-sensitive adhesive layer. This dissolution aid fills the action of enhancing the solubility of the steroidal antiphlogistic when it is directly dissolved in the pressure-sensitive adhesive.

Examples of the dissolution aid used in the present invention include crotamiton, ethanol, urea, water-soluble organic amines, propylene glycol fatty acid esters, 1-menthol and peppermint oil. The dissolution aids may be used either singly or in any combination thereof. Among these, crotamiton is preferred in that the effect of dissolution aid on the steroidal antiphlogistic is high, and a necessary level of pharmacological effect is easy to be achieved.

### (4) Accelerator for percutaneous absorption:

Examples of the accelerator for percutaneous absorption used in the present invention include polyhydric alcohol alkyl ethers, polyoxyethylene alkyl ethers, glycerides (i.e., glycerol fatty acid esters), polyhydric alcohol medium-chain fatty acid esters, alkyl lactates, dibasic acid alkyl esters, acylated amino acids, aliphatic alcohols and fatty acids.

These accelerators for percutaneous absorption may be used either singly or in any combination thereof. Among these, polyhydric alcohol alkyl ethers, polyoxyethylene alkyl ethers, glycerides and mixtures thereof are preferred in that the steroidal antiphlogistic is easy to achieve a necessary level of pharmacological effect.

Examples of the polyhydric alcohol alkyl ethers include alkyl ethers of polyhydric alcohols such as glycerol, ethylene glycol, propylene glycol, 1,3-butylene glycol, diglycerol, polyglycerol, diethylene glycol, polyethylene glycol, dipropylene glycol, polypropylene glycol, sorbitan, sorbitol, isosorbide, methylglucosides, oligosaccharides and reducing oligosaccharides. The number of carbon atoms in the alkyl group of the polyhydric alcohol alkyl ethers is preferably 6 to 20.

Among these, glycerol alkyl ethers are preferred, and glycerol alkyl ethers having the alkyl group of 12 to 18 carbon atoms are particularly preferred. Specific examples of such glycerol alkyl ethers include glycerol cetyl ether, glycerol stearyl ether and glycerol α-monoisostearyl ether.

The polyoxyethylene alkyl ethers are preferably polyoxyethylene alkyl ethers, the alkyl group of which has 6 to 20 carbon atoms, and the number of repeating units (-O-CH₂CH₂-) of the polyoxyethylene chain of which is 1 to 9. Specific examples of such polyoxyethylene alkyl ethers include polyoxyethylene lauryl ether, polyoxyethylene cetyl ether and polyoxyethylene stearyl ether.

The glycerides are preferably glycerol esters of fatty acids having 6 to 18 carbon atoms. The glycerides are divided into monoglycerides, diglycerides and triglycerides according to the number of fatty acids bonded thereto. However, any of them may be used. The glyceride may be a mixture (for example, a mixture of mono- and diglycerides) thereof.

Preferable examples of the fatty acid component forming the glyceride include caprylic acid (i.e., octanoic acid), capric acid (i.e., decanoic acid), lauric acid (i.e., dodecanoic acid), myristic acid (i.e., tetradecanoic acid), palmitic acid (i.e., hexadecanoic acid), stearic acid (i.e., octadecanoic acid) and oleic acid.

Preferable examples of the glycerides include caprylic acid glycerides, capric acid glycerides, lauric acid glycerides, myristic acid glycerides, palmitic acid glycerides, stearic acid glycerides and oleic acid glycerides. These glycerides include mono-, di- and triglycerides and mixtures thereof. Among these, mono-, di- and triglycerides of caprylic acid and mixtures thereof are particularly preferred. The glyceride is preferably used in combination with any other accelerator for percutaneous absorption from the viewpoint of percutaneous absorption.

### (5) Pressure-sensitive adhesive:

Examples of the pressure-sensitive adhesive used in the present invention include acrylic pressure-sensitive adhesives, rubber pressure-sensitive adhesives and silicone pressure-sensitive adhesives. Among these, rubber pressure-sensitive adhesives are preferred.

Examples of the rubber pressure-sensitive adhesives include those obtained by adding a tackifier such as a rosin resin, terpene resin, coumarone-indene resin or petroleum resin to a rubbery elastomer such as natural rubber, a styrene-isoprene-styrene block copolymer, polyisobutylene, polybutene or polyisoprene.

The rubber pressure-sensitive adhesives may contain a softening agent such as liquid polybutene, liquid polyisobutylene or mineral oil; a filler such as titanium oxide and zinc oxide; an antioxidant such as butylhydroxytoluene; and/or the like as needed.

Among the rubber pressure-sensitive adhesives, pressure-sensitive adhesives comprising, as a main base component, a styrene-isoprene-styrene block copolymer and obtained by blending it with a tackifier and optionally any other rubbery elastomer or the like are preferred from the viewpoint of percutaneous absorption and tackiness.

Examples of the acrylic pressure-sensitive adhesives include (co)polymers of at least one alkyl (meth)acrylate and copolymers of an alkyl (meth)acrylate and a functional monomer and/or vinyl ester monomer copolymerizable therewith. The functional monomer is used in a proportion of generally 0 to 30% by weight, preferably 2 to 10% by weight, while the vinyl ester monomer is used in a proportion of generally 0 to 40% by weight, preferably 5 to 30% by weight.

The alkyl group of the alkyl (meth)acrylate preferably has 4 to 10 carbon atoms. Particularly preferable examples of such an alkyl (meth)acrylate include butyl acrylate, octyl acrylate, 2-ethylhexyl acrylate, nonyl acrylate and isononyl acrylate. Examples of the functional monomer include (meth)acrylic acids having a functional group, and specific examples thereof include acrylic acid, methacrylic acid and 2-hydroxyethylacrylic acid. Examples of the vinyl ester monomer include vinyl acetate and vinyl laurate.

Examples of the silicone pressure-sensitive adhesives include those comprising a difunctional or trifunctional polysiloxane as a main component.

### (6) Proportions of individual components used:

The steroidal antiphlogistic is used in a proportion of generally 0.1 to 10 parts by weight, preferably 0.3 to 6 parts by weight, more preferably 0.5 to 3 parts by weight per 100 parts by weight of the pressure-sensitive adhesive. If the proportion of the steroidal antiphlogistic is too low, it is difficult to achieve a sufficient pharmacological effect. If the proportion is too high, crystals are easy to deposit.

The dissolution aid is used in a proportion of generally 0.2 to 20 parts by weight, preferably 0.6 to 12 parts by weight, more preferably 1 to 6 parts by weight per 100 parts by weight of the pressure-sensitive adhesive. If the proportion of the dissolution aid is too low, it is difficult to prepare a high-concentration solution of the steroidal antiphlogistic, the stability of the resulting preparation becomes insufficient, and so the steroidal antiphlogistic tends to deposit in the pressure-sensitive adhesive. If the proportion of the dissolution aid is too high, the effect of dissolution aid is saturated, and there is a possibility that the tackiness of the pressure-sensitive adhesive layer may be deteriorated.

The accelerator for percutaneous absorption is used in a proportion of generally 1 to 40 parts by weight, preferably 2 to 35 parts by weight, more preferably 3 to 30 parts by weight per 100 parts by weight of the pressure-sensitive adhesive. If the proportion of the accelerator for percutaneous absorption is too low, the permeability of the steroidal antiphlogistic through the skin becomes insufficient. If the proportion is too high, the tackiness of the pressure-sensitive adhesive layer is easy to be impaired.

The pressure-sensitive adhesive layer may contain various kinds of additives publicly known in this technical field as needed so far as the pharmacological effect of the steroidal antiphlogistic and the tackiness of the pressure-sensitive adhesive are impaired.

### (7) Preparation process of percutaneous absorption type steroid preparation for external use:

The percutaneous absorption type steroid preparation for external use according to the present invention is a patch obtained by providing a pressure-sensitive adhesive layer containing the steroidal antiphlogistic, dissolution aid and accelerator for percutaneous absorption on the support. Release paper is generally used on the pressure-sensitive adhesive layer. The release paper is used for the purpose of protecting the pressure-sensitive adhesive layer, and examples thereof include those obtained by subjecting one sides of polyethylene-coated woodfree paper, polyolefin-coated glassine paper, polyethylene terephthalate (polyester) films, polypropylene films, etc. to a silicone treatment.

The percutaneous absorption type steroid preparation for external use according to the present invention can be produced by a solvent type coating process, calender type coating process or the like which is a general production process of a patch. In the solvent type coating process, an organic solvent solution of a pressure-sensitive adhesive component is prepared, and the resultant pressure-sensitive adhesive solution is mixed with a steroidal antiphlogistic, a dissolution aid and an accelerator for percutaneous absorption to prepare a coating formulation. After this coating formulation is then applied on to release paper and dried, a support is stuck on the resultant pressure-sensitive adhesive layer. A process in which the coating formulation is applied to one side of the support and dried, and the release paper is then stuck on the resultant pressure-sensitive adhesive layer may also be adopted.

More specifically, in the solvent type coating process, an organic solvent such as n-hexane, toluene or ethyl acetate is placed in a metal-made pot lined with glass, to which a rubbery elastomer, a tackifier, an antioxidant, etc. are added, and the resultant mixture is stirred for about 2 to 10 hours, preferably about 3 to 7 hours until they are uniformly dissolved. Prescribed amounts of the steroidal antiphlogistic, dissolution aid and accelerator for percutaneous absorption are then added to this pressure-sensitive adhesive solution, and the resultant mixture is continuously stirred for about 10 to 120 minutes. A prescribed amount of the coating formulation obtained in such a manner is uniformly applied on to the support by means of a coater such as a knife coater, comma coater or reverse coater. After the application, the support is placed for about 1 to 10 minutes under an atmosphere controlled at a fixed temperature of 40 to 120°C to vaporize out the organic solvent. Suitable drying conditions are preset according to the kind of the organic solvent used and the thickness of the coating layer. Release paper such as a polyester film or polyethylene-coated woodfree paper subjected to a silicone treatment is stuck on the surface of the pressure-sensitive adhesive layer. The resultant sheet is cut into a prescribed size to provide a patch. The coating formulation may be applied to one side of the release paper to be transferred to the support.

In the case of the calender coating process, for example, a rubbery elastomer is masticated at 130°C for 20 minutes, the temperature is lowered to 120°C, and a petroleum tackifier is then added to knead the mixture for 10 minutes. After the temperature is lowered to 80°C, a softening agent is added, the resultant mixture is kneaded for 10 minutes, and a steroidal antiphlogistic, a dissolution aid and an accelerator for percutaneous absorption are lastly added to knead the resultant mixture for 10 minutes, thereby obtaining an agent-containing ointment. This product is cast in a thickness of 0.1 mm on a polyester film subjected to a silicone treatment, and a support is laminated on the pressure-sensitive adhesive layer, thereby obtaining a patch. The temperature, kneading time, etc. are suitably preset within respective preferable ranges according to the kind of a base material of the pressure-sensitive adhesive used, and the like.

### EXAMPLES

The present invention will hereinafter be described in more detail by the following Examples and Comparative Examples. However, the present invention is not limited only to these examples.

### [Example 1]

In a mixed solvent of hexane/toluene (weight ratio: 2/1) were dissolved 60 parts by weight of a styrene-isoprene-styrene block copolymer (JSR SIS5000, product of Japan Synthetic Rubber Co., Ltd.) and 40 parts by weight of polyisoprene rubber (KURAPRENE IR-10) as rubbery elastomers, 75 parts by weight of an alicyclic saturated hydrocarbon resin (Arkon P-100) as a tackifier and 60 parts by weight of liquid isoprene rubber (KURAPRENE LIR-30) as a softening agent to obtain a pressure-sensitive adhesive solution at a concentration of 40% by weight.

In 100 g of the resultant pressure-sensitive adhesive solution (pressure-sensitive adhesive component 40 g), were dissolved 0.4 g of prednisolone 21-(2E,6E-farnesylate), 0.8 g of crotamiton and 6 g of polyoxyethylene lauryl ether [the number of repeating units (-O-CH₂CH₂-) of the polyoxyethylene chain: 1] to obtain a coating formulation. This coating formulation was applied to release paper so as to give a dry thickness of 50 µm. After drying the coating formulation, a nonwoven polyester fabric (7850, product of Shinwa K.K.) was stuck as a support on the resultant pressure-sensitive adhesive layer to provide a patch.

### [Example 2]

A process was carried out under the same conditions as in Example 1 except that 0.4 g of prednisolone 21-(2E,6E-farnesylate), 2 g of crotamiton and 2 g of glycerol α-monoisostearyl ether were dissolved in 100 g of the pressure-sensitive adhesive solution prepared in Example 1, thereby obtaining a patch.

### [Example 3]

A process was carried out under the same conditions as in Example 1 except that 0.4 g of prednisolone 21-(2E,6E-farnesylate), 2 g of crotamiton, 10 g of polyoxyethylene lauryl ether [the number of repeating units (-O-CH₂CH₂-) of the polyoxyethylene chain: 1] and 2 g of caprylic acid glyceride were dissolved in 100 g of the pressure-sensitive adhesive solution prepared in Example 1, thereby obtaining a patch. Homotex PT (trademark; product of Kao Corporation) which is a mixture of caprylic acid monoglyceride (about 55% by weight) and caprylic acid diglyceride (about 45% by weight) was used as the caprylic acid glyceride.

### [Referential Example 1]

Farnezone gel (trademark) [active component: prednisolone 21-(2E,6E-farnesylate) 1.4%] which is a gel ointment containing a steroidal antiphlogistic marketed by TAIHO PHARMACEUTICAL CO., LTD. was used for the sake of reference.

### [Comparative Example 1]

A process was carried out under the same conditions as in Example 1 except that only 0.4 g of prednisolone 21-(2E,6E-farnesylate) were dissolved in 100 g of the pressure-sensitive adhesive solution prepared in Example 1, and neither crotamiton nor polyoxyethylene lauryl ether was added, thereby obtaining a patch.

### [Test Example] Skin-Permeability Test

After abdominal hair of hairless rats were shaved by means of an electric shaver under anesthesia with pentobarbital, the skins were taken out. Each of the skins was installed in a vertical diffusion cell. Hot water at a temperature of 32°C was circulated through the double structure cell to keep the interior of the cell under the constant temperature conditions. Each of test preparations of Examples 1 to 3, Referential Example 1 and Comparative Example 1 was administered on the keratin layer side of the skin.

Propylene glycol was added to a receiver to conduct sampling with time. Methanol was added to a sampled specimen, and the mixture was stirred and then centrifuged to remove proteins. The resultant solution was analyzed by high performance liquid chromatography (HPLC) to determine prednisolone 21-(2E,6E-farnesylate) permeated through the skin. To the receiver after the sampling was added propylene glycol in the same amount as the quantity sampled. The permeability of prednisolone 21-(2E,6E-farnesylate) in the respective test preparations through the skin were compared by the cumulative amount permeated after 48 hours. The measured results are shown in Table 1.

**Table 1**

| | | Ex. 1 | Ex. 2 | Ex. 3 | Ref. Ex. 1 | Comp. Ex. 1 |
|---|---|---|---|---|---|---|
| Cumulative amount of PNF¹⁾ permeated after 48 hours (µg/cm²) | Mean value²⁾ | 0.63 | 0.74 | 0.97 | 0.58 | 0.03 |
| | Standard deviation | 0.09 | 0.03 | 0.12 | 0.05 | 0.01 |

| | | | | | | |
|---|---|---|---|---|---|---|
| (Note) 1) PNF: prednisolone 21-(2E,6E-farnesylate). | | | | | | |
| 2) Mean value: a mean value at the number of measurements of 4 (n = 4). | | | | | | |

As apparent from the results shown in Table 1, it is understood that the patches (Examples 1 to 3) comprising the dissolution aid and accelerator for percutaneous absorption exhibit permeability of the steroidal antiphlogistic through the skin substantially equivalent or superior to the commercially available gel ointment (Referential Example 1), and so percutaneous absorption sufficient to treat can be achieved. In the case of the patch (Comparative Example 1) containing neither dissolution aid nor accelerator for percutaneous absorption, on the other hand, the permeability of the agent through the skin is markedly deteriorated, and so no satisfactory pharmacological effect can be achieved.

The patches of Examples 1 to 3 can be simply peeled from the surface of the skin if a side effect manifests. On the other hand, the commercially available gel ointment (Referential Example 1) is difficult to simply peel when applied to the surface of the skin once even if a side effect manifests.

### INDUSTRIAL APPLICABILITY

According to the present invention, there can be provided percutaneous absorption type steroid preparations for external use which contain a steroidal antiphlogistic, can precisely control a dose of the steroidal antiphlogistic, are good in percutaneous absorption and can easily avoid a side effect if develops after application.

## Claims

1. A percutaneous absorption type steroid preparation for external use comprising a steroidal antiphlogistic, wherein
(1) the preparation form of the preparation for external use is a patch that a pressure-sensitive adhesive layer containing the steroidal antiphlogistic is provided on a support, and
(2) the pressure-sensitive adhesive layer further contains a dissolution aid for the steroidal antiphlogistic and an accelerator for percutaneous absorption.

2. The percutaneous absorption type steroid preparation for external use according to Claim 1, wherein the steroidal antiphlogistic is a steroidal antiphlogistic selected from the group consisting of cortisone, hydrocortisone, prednisolone, methylprednisolone, triamcinolone, dexamethasone, betamethasone, paramethasone, and derivatives and salts thereof.

3. The percutaneous absorption type steroid preparation for external use according to Claim 1, wherein the steroidal antiphlogistic is prednisolone, a prednisolone derivative or a salt thereof.

4. The percutaneous absorption type steroid preparation for external use according to Claim 3, wherein the prednisolone derivative is 11β,17α,21-trihydroxy-1,4-pregnadiene-3,20-dione 21-[(E,E)-3,7,11-trimethyl-2,6,10-dodecatrienoate.

5. The percutaneous absorption type steroid preparation for external use according to Claim 1, wherein the dissolution aid is crotamiton, ethanol, urea, a water-soluble organic amine, a propylene glycol fatty acid ester, 1-menthol, peppermint oil or a mixture of at least two thereof.

6. The percutaneous absorption type steroid preparation for external use according to Claim 1, wherein the dissolution aid is crotamiton.

7. The percutaneous absorption type steroid preparation for external use according to Claim 1, wherein the accelerator for percutaneous absorption is a polyhydric alcohol alkyl ether, a polyoxyethylene alkyl ether, a glyceride, a polyhydric alcohol medium-chain fatty acid ester, an alkyl lactate, a dibasic acid alkyl ester, an acylated amino acid, an aliphatic alcohol, a fatty acid or a mixture of at least two thereof.

8. The percutaneous absorption type steroid preparation for external use according to Claim 7, wherein the polyhydric alcohol alkyl ether is a glycerol alkyl ether.

9. The percutaneous absorption type steroid preparation for external use according to Claim 7, wherein the polyoxyethylene alkyl ether is a polyoxyethylene alkyl ether, the alkyl group of which has 6 to 20 carbon atoms.

10. The percutaneous absorption type steroid preparation for external use according to Claim 9, wherein the polyoxyethylene alkyl ether is a polyoxyethylene alkyl ether, the number of repeating units (-O-CH₂CH₂-) of the polyoxyethylene chain of which is 1 to 9.

11. The percutaneous absorption type steroid preparation for external use according to Claim 7, wherein the glyceride is a glycerol ester of a fatty acid having 6 to 18 carbon atoms.

12. The percutaneous absorption type steroid preparation for external use according to Claim 1, wherein the pressure-sensitive adhesive layer contains, per 100 parts by weight of the pressure-sensitive adhesive, 0.1 to 10 parts by weight of the steroidal antiphlogistic, 0.2 to 20 parts by weight of the dissolution aid and 1 to 40 parts by weight of the accelerator for percutaneous absorption.

13. The percutaneous absorption type steroid preparation for external use according to Claim 1, wherein the pressure-sensitive adhesive is a rubber pressure-sensitive adhesive.

14. The percutaneous absorption type steroid preparation for external use according to Claim 13, wherein the rubber pressure-sensitive adhesive comprises a styrene-isoprene-styrene block copolymer as a base material.
